# FASCICULE DE BREVET EUROPEEN

(11) **EP 1 807 084 B1**
(45) Date de publication et mention de la délivrance du brevet: **15.12.2010**
(21) Numéro de dépôt: 05804722.6
(22) Date de dépôt: 14.10.2005
(51) Int. Cl.: A61K 31/4985, A61P 11/00, A61P 1/00

(54) **UTILISATION DE DERIVES DE PYRROLO-PYRAZINES POUR LE TRAITEMENT DE LA MUCOVISCIDOSE**
VERWENDUNG VON PYRROLO-PYRAZINDERIVATEN ZUR BEHANDLUNG DER ZYSTISCHEN FIBROSE
USE OF PYRROLOPYRAZINE DERIVATIVES FOR THE PRODUCTION OF MEDICAMENTS FOR THE TREATMENT OF MUCOVISCIDOSIS

(30) Priorité: 15.10.2004 FR 0410962
(43) Date de publication de la demande: 18.07.2007
(73) Titulaire: CENTRE NATIONAL DE LA RECHERCHE SCIENTIFIQUE (CNRS), 75794 Paris Cedex 16 (FR); UNIVERSITE DE POITIERS, 86034 Poitiers Cedex (FR)
(72) Inventeur: BECQ, Frédéric, F-86280 POITIERS (FR); MEIJER, Laurent, F-29680 ROSCOFF (FR); METTEY, Yvette, F-94300 Vincennes (FR)
(74) Mandataire: Peaucelle, Chantal
(86) Numéro de dépôt international: PCT/FR2005/002558
(87) Numéro de publication internationale: WO 2006/042950

(56) Documents cités:
- EP-A- 1 388 541
- WO-A-03/089434
- WO-A-2004/032874
- METTEY Y ET AL: "Aloisines, a new family of CDK/GSK-3 Inhibitors. SAR Study, crystal structure in complex with CDK2, enzyme selectivity, and cellular effects" JOURNAL OF MEDICINAL CHEMISTRY, AMERICAN CHEMICAL SOCIETY. WASHINGTON, US, vol. 46, no. 2, 18 décembre 2002 (2002-12-18), pages 222-236, XP002285187 ISSN: 0022-2623
- MEIJER L ET AL: "Pharmacological inhibitors of glycogen synthase kinase 3" TRENDS IN PHARMACOLOGICAL SCIENCES 2004 UNITED KINGDOM, vol. 25, no. 9, 2004, pages 471-480, XP002326405 ISSN: 0165-6147
- KUNICK CONRAD ET AL: "1-Azakenpaullone is a selective inhibitor of glycogen synthase kinase-3 beta." BIOORGANIC & MEDICINAL CHEMISTRY LETTERS. 19 JAN 2004, vol. 14, no. 2, 19 janvier 2004 (2004-01-19), pages 413-416, XP002326403 ISSN: 0960-894X
- DOBLE B W ET AL: "GSK-3: Tricks of the trade for a multi-tasking kinase" JOURNAL OF CELL SCIENCE 01 APR 2003 UNITED KINGDOM, vol. 116, no. 7, 1 avril 2003 (2003-04-01), pages 1175-1186, XP002326404 ISSN: 0021-9533

## Description

L'invention vise l'utilisation de dérivés de pyrrolo-pyrazines pour fabriquer des médicaments capables de restaurer l'adressage de protéines du réticulum endoplasmique vers les membranes plasmiques. Elle vise tout particulièrement le traitement de la mucoviscidose.

La mucoviscidose (CF: *Cystic Fibrosis*) est la maladie génétique récessive, autosomique, létale la plus répandue dans les populations européennes et nord américaines. Le gène CF blocus 7q31) code pour la protéine trans-membranaire nommée CFTR (pour *Cystic Fibrosis Transmembrane Conductance Regulator*). Des mutations du gène CF provoquent un transport anormal d'eau et d'électrolytes à travers les membranes cellulaires de divers organes comme les poumons, les glandes sudoripares, l'intestin et le pancréas exocrine. Bien qu'ils existe plus de 1000 mutations de la protéine CFTR, la mutation la plus fréquente (70 % des patients) est la délétion d'une phénylalanine dans le domaine NBF1 en position 508 (delF508). La principale cause de mortalité des patients CF est liée à cette délétion et conduit à des infections ou à une insuffisance pulmonaire provoquées par une augmentation de la viscosité du mucus. Cette viscosité entraîne l'occlusion des voies respiratoires et favorise les infections par les bactéries opportunistes. Une aggravation est de plus constatée au niveau digestif et notamment pancréatique (patient pancréatique-insuffisant). La protéine CFTR est une glycoprotéine de 1480 acides aminés, appartenant à la superfamille des transporteurs membranaires ABC. CFTR est un canal chlorure localisé dans la membrane plasmique apicale des cellules épithéliales pulmonaires chez les individus sains.

CFTR est responsable du transport trans-épithélial d'eau et d'électrolytes et permet chez un individu sain l'hydratation des voies aériennes pulmonaires.

Chez les patients CF, homozygotes pour la mutation delF508, et plus généralement pour les mutants de classe II (mutations produisant une protéine absente de la membrane plasmique), cette protéine est absente des membranes plasmiques du fait d'un mauvais adressage de la protéine qui est retenue dans le réticulum endoplasmique (RE). L'hydratation des voies aériennes pulmonaires n'est plus fonctionnelle dans ce cas. La délétion delF508 perturbe le repliement du domaine NBF1 et empêche la maturation complète de la protéine qui est donc dégradée très tôt au cours de sa biosynthèse. Cependant, si la protéine delF508 atteint la membrane, elle fonctionne comme un canal chlorure.

Une des clés d'un traitement de cette maladie consiste donc en un ré-adressage de delF508 vers la membrane plasmique des cellules au niveau de laquelle l'activité de transport de delF508 peut être stimulée par des agonistes physiologiques.

WO 2004/032874 au nom de Scios Inc et al se rapporte à des dérivés d'azaindole possédant des propriétés inhibitrices de la kinase p38 et à ce titre sont retenus pour le traitement de diverse maladies.

Mettey et al décrivent dans J.Med.Chem.2003, 46,222-235 des aloisines en tant qu'inhibiteurs de CDK/GSK-3 et utilisables comme agents anti-polifératifs.

EP 1 388 541 concerne des pyrrolopyrazines présentant des propriétés inhibitrices de kinoses leur conférant un intérêt notamment dans la maladie d'Alzheimer et de Parkinson.

De manière surprenante, les inventeurs ont mis en évidence que des dérivés notamment connus pour leur effet anti-prolifératif étaient capables d'activer le CFTR sauvage et des formes mutées et de provoquer une re-localisation membranaire de la protéine delF508-CFTR restaurant ainsi sa capacité de transport trans-membranaire. De plus, ces dérivés présentent l'intérêt d'une innocuité élevée.

L'invention à donc pour but de fournir une nouvelle utilisation de ces dérivés pour fabriquer des médicaments pour le traitement de la mucoviscidose.

Les dérivés utilisés conformément à l'invention répondent à la formule (I): 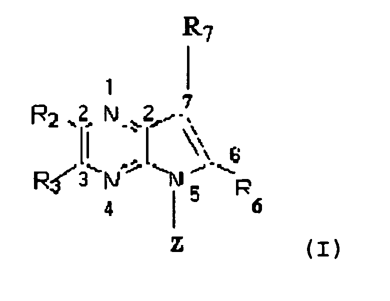 dans laquelle
- R2 et R3 sont identiques ou différents et représentent H, C1-C6 alkyl, ledit alkyl étant une chaîne alkyle droite ou ramifiée, le cas échéant substituée,
- R6 est un cycle aromatique Ar ou un cycloalkyle, le cas échéant substitué, ledit cycloalkyle étant le cas échéant substitué par un groupe aryle qui peut être également substitué,
- R7 est H, C1-C6 alkyl, (alk.)ₙ-hal., CH₂-CH = CH₂, CH₂-cycloalkyl, CH₂-Ar,
- Z est H ou CH₃.

De préférence, R2 et R3, et/ou Z et/ou R7 sont différents de H.

Ar est de préférence un phényl, naphtyl, furyl, thiényl, pyridyl, cyclopropyl phényl, phényl dioxolyl.

"Cycloalkyl" est un C3-C6 cycloalkyl.

Les substitutions du groupe alkyl, du cycle aromatique ou cycloalkyl sont choisies dans le groupe comprenant un ou plusieurs halogènes (F,Cl, Br, I, CF₃), OH, NH₂, N(H, alkyl), N(alkyl)₂, O-alkyl, COOH,COO-alkyl, CONH₂, CON(H,alkyl), CON(alkyl)₂, NHCONH₂, NHCON(H,alkyl), NHCON(alkyl)₂, N(alkyl)CONH_{2.} N(alkyl)CON(H,alkyl), N(alkyl)CON(alkyl)₂, alkoxy, CN, O-SO₂-NH₂, O-SO₂-N(H,alkyl),-O-SO₂-N (alkyl)₂, SH,S-alkyl. Un ou plusieurs substituants peuvent être présents.

"Alkyl" est un C1-C6 alkyl et comprend les isomères.

"Alkoxy" comprend un groupe C1-C6 alkyl.

"Alk." est un groupe C1-C6 alkylène, n est 1-6, et "hal." est F, Cl, Br, I or CF₃.

Lesdits pyrrolo [2,3-b] pyrazines, également désignés par aloisines ci-après, sont capables de restaurer l'adressage de la protéine CFTR vers les membranes plasmiques des cellules et constituent donc des composés de grand intérêt pour le traitement de la mucoviscidose liée à de tels problèmes de défaut d'adressage.

Comme illustré par les exemples, ils sont particulièrement efficaces pour provoquer la re-localisation de la protéine delF508-CFTR dan la mucoviscidose où cette protéine est retenue dans le réticulum endoplasmique, et de restaurer ainsi sa capacité de transfert trans-membranaire.

Des dérivés préférés de pyrrolo-pyrazines présentent la formule (II): 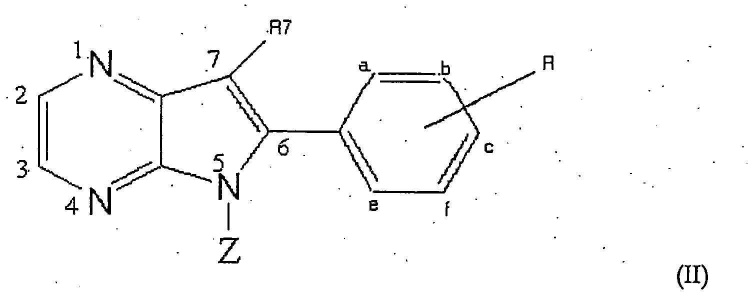 dans laquelle
- le groupe phényl en position 6 est substitué par un, deux ou trois substituants R choisis dans le groupe comprenant :
- H, -OH, alkyl, -O alkyl, hal., -NH₂, -N(H,alkyl), - N(alkyl)₂, -O-SO₂-NH₂, -O-SO₂-N(H, alkyl), -O-SO₂-N(alkyl)₂, - COOH, -COO-alkyl, CONH₂, -CON(H,alkyl), -CON(alkyl)₂,
- R7 est H, alkyl, (al.)ₙ hal., -CH₂-CH = CH₂,(alk.)n-cycloalkyl, alk.-Ar, et
- Z est H ou CH₃.

Dans un groupe préféré, Z et/ou R7 sont différents de H.

Un composé tout particulièrement préféré correspond à l'aloïsine A répondant à la formule (III)

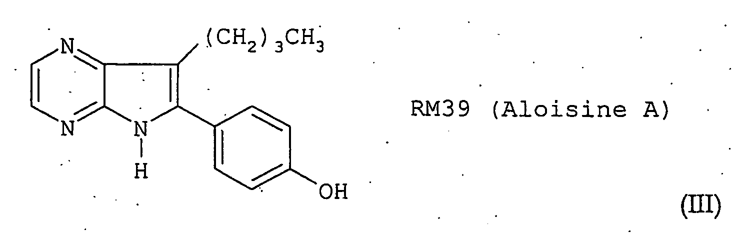

Lors de l'élaboration des médicaments, les' principes actifs, utilisés en quantités thérapeutiquement efficaces, sont mélangés avec les véhicules pharmaceutiquement acceptables pour le morde d'administration choisi. Ces véhicules peuvent être solides ou liquides.

Ainsi pour une administration par voie orale, les médicaments préparés sous forme de gélules, comprimés, dragées, capsules, pilules, gouttes, sirops et analogues. De tels médicaments peuvent renfermer de 1 à 100 mg de principe actif par unité.

Pour l'administration par voie injectable (intraveineuse, sous-cutanée, intramusculaire), les médicaments se présentent sous forme de solutions stériles ou stérilisables.

Ils peuvent être également sous forme d'émulsions ou de suspensions.

Les médicaments de l'invention sont plus particulièrement administrés sous forme d'aérosols.

Les doses par unité de prise peuvent varier de 1 à 50 mg de principe actif. La posologie quotidienne est choisie de manière à obtenir une concentration finale d'au plus 100 µM en dérivé de pyrrolo-pyrazines dans le sang du patient traité.

D'autres caractéristiques et avantages de l'invention seront donnés dans les résultats rapportés ci-après afin d'illustrer l'invention.

Dans ces exemples, il est fait référence aux figures 1 à 7 qui représentent, respectivement :
- les figures 1A à 1C, la formule de l'aloïsine A, l'activation du canal chlorure CFTR par l'aloïsine A sur des cellules CHO (fig.1B) et sur des cellules épithéliales pulmonaires humaines Calu-3 (fig.1C) ;
- le figures 2A et 2B, l'activation par l'aloïsine A de la protéine G551D-CFTR dans les cellules CHO (fig. 2A) et la protéine delF508 dans les cellules épithéliales humaines pulmonaires de lignée CF15 (fig.2B) ;
- les figures 3A et 3B, l'EC₅₀ de l'aloïsine A à 50µM (fig.3A) et l'effet d'inhibiteurs de CFTR sur l'activité de delF508 après traitement par l'aloïsine A ;
- la figure 4, la localisation de delF508 chez des patients CF et son re-adressage vers la membrane après traitement par l'aloïsine A ;
- les figures 5A et 5B, les tests de toxicité de l'aloïsine A sur des cellules CHO-WT après incubation de 2 h (fig. 5A) et de 24 h (fig.5B),
- les figures 6A à 6C, l'immunolocalisation de delF508-CFTR après 2 h de traitement avec l'aloïsine A,
- la figure 7, l'activation de delF508-CFTR dans les cellules CF15 après traitement avec l'aloïsine A.

### MATERIEL ET METHODES

### M1. Culture cellulaire

Cellules CHO-WT : Les cellules CHO (Chinese Hamster Ovary) sont des fibroblastes qui ont été transfectés avec le gène du CFTR sauvage (CFTR-WT). Ces cellules vont donc surexprimer la protéine CFTR.

Milieu de culture Milieu MEM alpha (GIBCO) + 7 % de sérum de veau foetal + 0,5 % de pénicilline/streptomycine + 100 µM de.méthotrexate (améthoptérine, Sigma).

Cellules CF15 : Les cellules CF15 sont des cellules épithéliales humaines d'origine nasale qui expriment le gène ΔF508-CFTR.

Milieu de culture : Milieu DMEM + HAM F12 + 10 % de FCS + 0,6 % de pénicilline/streptomycine + facteurs de croissance (insuline 5 µg/ml, transferrine 5 µg/ml, épinéphrine 5,5 µM, adénine 0,18 mM, EGF 10 ng/ml, T3 2 nM, hydrocortisone 1,1 µM).

Cellules Calu-3 : Les cellules Calu-3 sont des cellules épithéliales humaines d'origine- pulmonaire qui expriment le gène du CFTR sauvage .

Milieu de culture : Milieu DMEM/F12 avec glutamax + 7 % de sérum de veau foetal + 1 % de pénicilline/streptomycine.

### M2. Immunomarquage

L'immunomarquage permet de visualiser la localisation cellulaire de la protéine CFTR grâce à un anticorps (Ac) primaire anti-CFTR, puis un anticorps secondaire anti-anticorps primaire marqué au fluorophore Cy3.

lets cellules sont préalablement ensemencées sur des lamelles dans du milieu de culture approprié. 3 lavages au TBS (NaCl : 157 mM, Tris base : 20 µM, pH 7,4) de 5 min chacun sont effectués. Les cellules sont alors fixées par ajout de TBS-paraformaldéhyde (3 %) pendant 20 min. Après 3 lavages au TBS (5 min), les cellules sont incubées avec du TBS-triton 0,1% (10 min) qui permet la formation de trous dans la membrane cellulaire, puis 3 lavages au TBS sont à nouveau effectués avant la mise en présence des cellules avec le TBS-BSA 0,5 %-saponine 0,05 % pendant 1 h. Les cellules sont ensuite incubées avec l'anticorps primaire anti-C terminal CFTR (2 µg/ml) pendant 1 h. 3 lavages au TBS-BSA-saponine de 5 min chacun sont réalisés avant l'incubation avec l'anticorps secondaire GAM-cy3 (1/400) pendant 1 h. Suite à 2 lavages au TBS de 5 min, les noyaux sont marqués par incubation au Topro3 (1/1000) pendant 5 min. Enfin, les lamelles peuvent être montées sur lame après 3 derniers lavages au TBS de 5 min. Les lames sont observées au microscope confocal (Bio-Rad) grâce à une excitation au laser aux longueurs d'onde appropriées. Afin de différencier le marquage entre Cy3 et Topro3 la couleur de fluorescence de Topro3 a été changée en bleu (couleur des noyaux).

### M3. Efflux de radiotraceurs

Les mesures de transport d'ions chlorure dans les cellules ont été réalisées à l'aide de la technique des efflux d'iodure radioactif (Becq et al., 1999 ; Dormer et al., 2001). Le traceur (¹²⁵I) est incorporé dans le milieu intracellulaire. Puis, la quantité de radiotraceur qui sort de la cellule est comptée après l'ajout de différents agents pharmacologiques. L'iodure est utilisé comme un traceur du transport d'ions chlorure. ¹²⁵I a de plus l'avantage d'avoir une courte durée de vie comparée à celle d'autres marqueurs comme ³⁶Cl (1/2 vie respective : 30 jours et 300 000 ans).

Les cellules sont mises en culture sur des plaques 24 puits dans un milieu adéquat. 2 rinçages avec du milieu d'efflux (NaCl : 136,6 mM, KCl : 5,4 mM, KH₂PO₄ : 0,3 mM, NaH₂PO₄ : 0,3 mM, NaHCO₃ : 4,2 mM, CaCl₂ 1,3 mM; MgCl₂: 0,5 mM, MgSO₄ : 0, 4 mM, HEPES : 10 mM, D-glucose : 5, 6 mM) sont effectués afin d'éliminer les cellules mortes qui relarguent la radioactivité de façon anarchique. Puis, les cellules sont mises à incuber avec 500 µl de charge (1 µCi/ml de ¹²⁵INa) pendant 30 min pour les CHO-WT ou 1 h pour les CF15 et Calu-3. L'iodure s'équilibre de part et d'autre de la membrane cellulaire. Le robot (MultiPROBE, Packard) réalise les étapes suivantes : le milieu de charge est rincé avec du milieu d'efflux pour éliminer la radioactivité extracellulaire. Le surnageant est collecté toutes les minutes dans des tubes à hémolyse et le milieu est remplacé par un volume équivalent (500 µl). Les prélèvements des 3 premières minutes ne subissent pas l'addition de drogue, ils permettent d'obvenir une ligne de base stable, caractérisant la sortie passive des ions I. Les 7 prélèvements suivants sont obtenus en présence de la molécule à tester. A la fin de l'expérience, les cellules sont lysées par ajout de 500 µl de NaOH (0,1 N)/0,1 % SDS (30 min), ainsi, la radioactivité restée à l'intérieur de la cellule peut être déterminée. La radioactivité présente dans les tubes à hémolyse est comptée en coups par minute (cpm) grâce à un compteur gamma (Cobra II, Packard). Les résultats en cpm sont exprimés sous forme de vitesse de sortie d'iodure radioactif (R) d'après la formule suivante : R (min⁻¹)=[In(¹²⁵I t₁)-In(¹²⁵I t₂)]/(t₁-t₂) avec ¹²⁵I t₁ : cpm au temps t₁ ; ¹²⁵I t₂ : cpm au temps t₂. Ce flux d'iodure est représenté sous forme de courbe. Afin de quantifier la sortie d'iodure due à l'administration de la molécule testée, on calcule le flux relatif suivant qui permet de s'affranchir du flux de base : Vitesse relative (min⁻¹) =Rpic-Rbasal. Enfin, ces résultats sont normalisés pour pouvoir comparer l'effet de différentes drogues entre elles. Les résultats sont présentés sous la forme de moyenne +/- SEM. Le test statistique de Student est utilisé pour comparer l'effet des drogues aux contrôles (les valeurs correspondantes à P<0,01 sont considérées comme statistiquement significatives).

### M4. Test de cytotoxicité

Le test de toxicité au MTT est un test colorimétrique qui repose sur la capacité des déshydrogénases mitochondriales à métaboliser le MTT (sel de tétrazolium jaune) en formazan (pourpre). L'absorbance, proportionnelle à la concentration de colorant convertit peut être alors mesurée par spectrophotométrie. Les cellules sont mises à incuber sur des plaques 96 puits en présence de l'agent à tester pendant 2 h. 3 contrôles sont réalisés : 100 % cellules vivantes : cellules sans agent.; 0 % cellules vivants : cellules laissées à l'air libre ; blanc : milieu sans cellule. Les cellules sont rincés avec du milieu RPMI sans rouge de phénol pour que la couler du milieu n'interfère pas dans les mesures de l'absorbance. Puis, elles sont incubées pendant 4 h avec 100 µl de solution de RPMI supplémentée en MTT (0,5 mg/ml). Le milieu est alors éliminé, l'ajout de 100 µl de DMSO permet de solubiliser le colorant converti (formazan). L'absorbance est mesurée par spectrophotométrie à 570 nm (pourpre) ; 630 nm (bruit de fond). Afin de s'affranchir du bruit de fond, le calcul suivant est effectué : DO_{réelle}=DO₅₇₀ₙₘ-DO₆₃₀ₙₘ. Puis, les résultats sont normalisés par rapport aux contrôles (100 % et 0 % de cellule vivantes) et sont présentés sous forme de moyenne +/-SEM.

### RESULTATS

### R1. L'aloïsine A active CFTR sauvage (wt), G551D et delF508

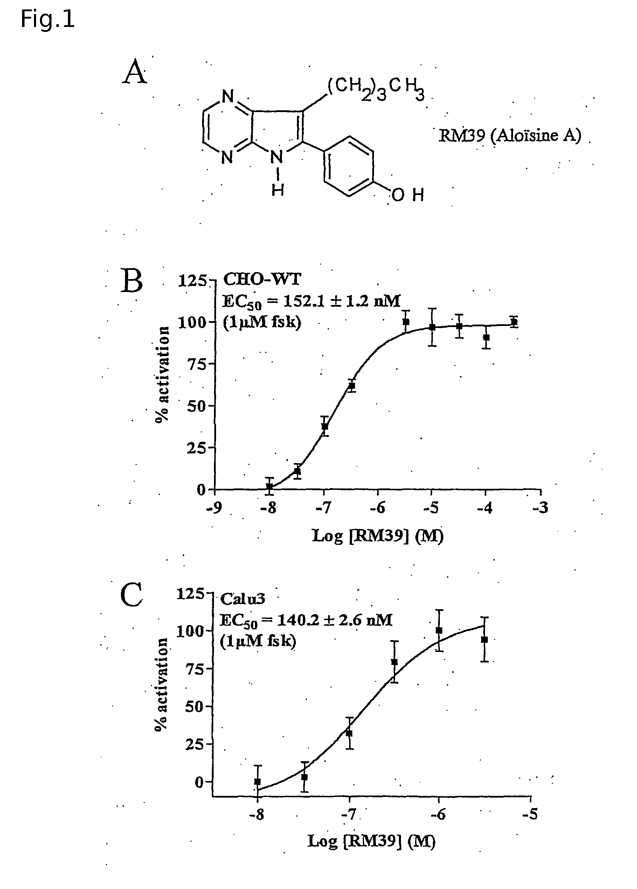

Une première série d'expérimentations montre que l'aloïsine A est capable d'activer le canal chlorure CFTR dans les cellules CHO (EC₅₀ = 152,1 nM, figure 1A) et dans les cellules épithéliales pulmonaires humaines de lignée Calu-3 (EC₅₀ = 140,1 nM, figure 1B).

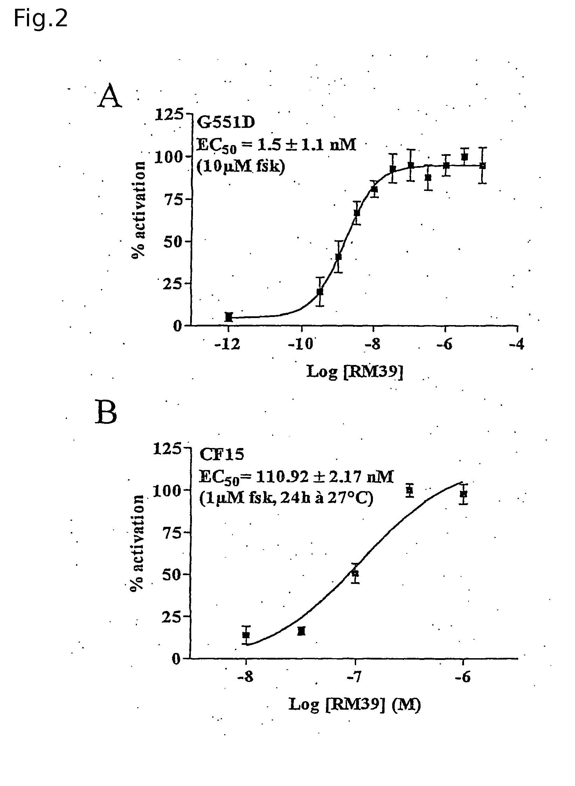

Deux formes mutées de CFTR ont été également testées. Il s'agit du mutant G551D et de delF508. La figure 2 montre que l'aloïsine A active la protéine G551D-CFTR dans la cellule CHO (EC₅₀ = 1,5 nM, figure 2A) et la protéine delF508 (EC₅₀ = 110 nM, figure 2B) dans les cellules épithéliales humaines pulmonaires de lignée CF15 après incubation des cellules à 27°C (une procédure qui permet de relocaliser la protéine delF508 vers la membrane).

Ces résultats démontrent que cette molécule est un activateur de CFTR sauvage, G551D et delF508.

### R2. Effet de l'aloïsine A sur l'adressage de delF508 dans les cellules CF15

L'étude de l'adressage de la protéine delF508-CFTR a été réalisée en combinant des approches de pharmacologie, d'imagerie cellulaire, des tests biochimiques et électrophysiologiques sur des cellules CF15 épithéliales humaines pulmonaires homozygotes pour la délétion delF508.

Pour chaque expérience, l'addition d'un cocktail (Forskoline 10 µM + Génistéine 30 µM) permet l'activation du CFTR quand celui-ci est fixé à la membrane. Ainsi, un efflux d'iodure pourra être observé si l'adressage de delF508 a été restauré.

Les résultats, présentés sous forme d'histogramme ont été normalisés par rapport à un traitement de référence (traitement des cellules au MPB-91 250 µM pendant 2 h) pour lequel on considère qu'on a 100 % d'activité CFTR.

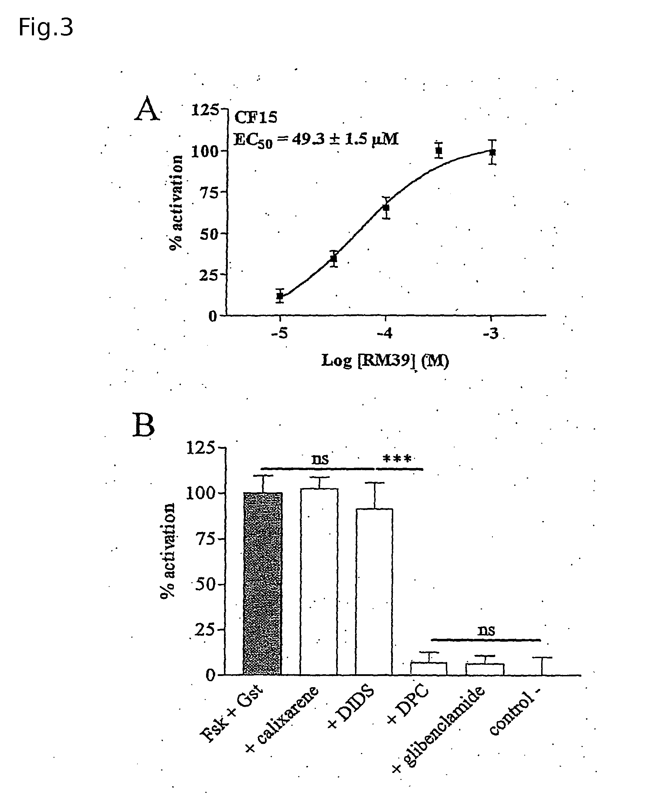

La figure 3A montre qu'après un traitement de 2 h avec 100 µM d'aloïsine A l'activité delF508-CFTR est restaurée.

Ces résultats démontrent ainsi que le traitement par l'aloïsine A des cellules CF15 pendant 2 h à 37°C restaure un adressage de la protéine delF508 et permet à celle-ci de fonctionner en tant que transporteur ionique.

En l'absence de traitement des cellules, la protéine delF508 n'est pas membranaire et il n'y a pas d'efflux d'iodure stimulé par le cocktail (Forskoline 10 µM, Génistéine 30 µM). L'EC₅₀ (concentration de la molécule qui donne 50 % de l'efficacité maximale) de l'aloïsine A a été déterminé à 50 µM (figure 3A, n=4, pour chaque condition). Pour préciser l'effet observé des inhibiteurs connus de CFTR sur l'activité de delF508 ont été testés après traitement par l'aloïsine A. Les résultats présentés figure 3B montrent que ce transport est bloqué par le glibenclamide et le DPC mais insensible au DIS et au calixarène. Ce profil pharmacologique correspond à celui de CFTR.

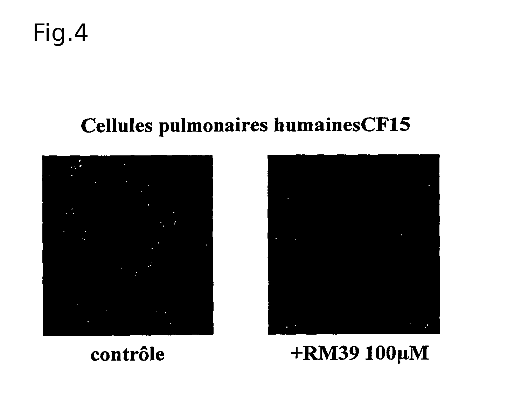

Chez les patients CF, la protéine delF508 est absente des membranes plasmiques du fait' d'un mauvais adressage de la protéine qui est retenue dans le réticulum endoplasmique (RE). Par imagerie cellulaire dans les cellules CF15, selF508 est effectivement localisée dans des compartiments intracellulaires (figure 4). En revanche le traitement avec 100 µM d'aloïsine A permet de rediriger la protéine delF508 vers la membrane comme le présente la figure 4.

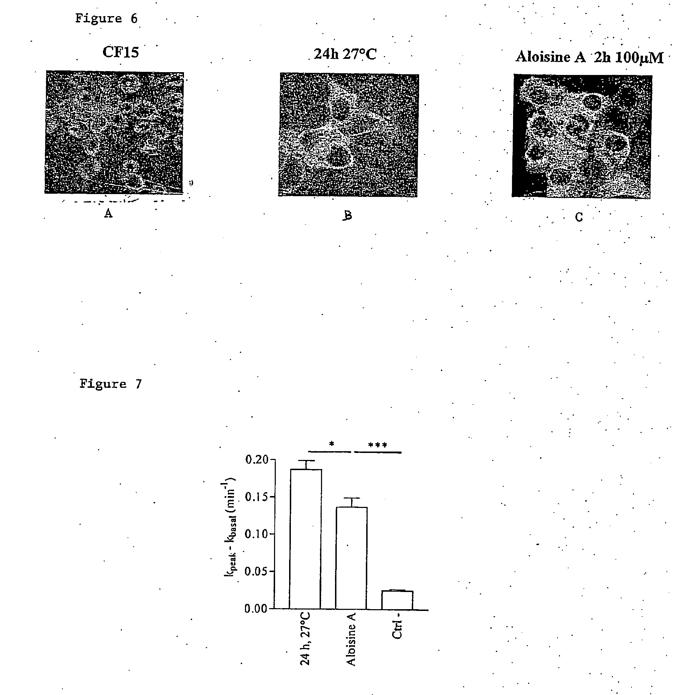

L'immunolocalisation de delF508-CFTR dans des cellules CF15 est également représentée sur les figures 6A à 6C qui montrent respectivement, la figure 6A : la visualisation confocale de CFTR-delF508 dans des cellules CF15 avec un anticorps monoclonal anti-CFTR de souris ; la figure 6B : les cellules CF15 traitées 24 h à 27°C, utilisées comme témoin positif ; la figure 6C : les cellules CF15 traitées 2 h avec de l'aloïsine A (100µM).

Sur la figure 7, on a représenté l'activation de delF508-CFTR dans les cellules CF15 après traitement avec l'aloïsine A.

Les efflux d'iodure ont été observés après 2 h d'incubation avec ces composés ou en l'absence de traitement.

Les cellules CF15 ayant subi un traitement 24 h à 27°C ont été utilisées comme contrôle positif et les cellules CF15 non traitées comme contrôle négatif (37°C).

Le tableau ci-dessous donne un résumé d'expériences de compétition réalisées par la technique d'efflux d'iodure enture l'aloïsine A et la machinerie de la chaperone du RE.

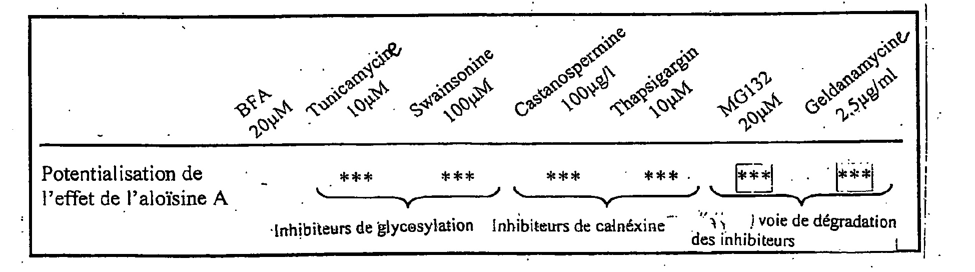

- inhibition, **P <0.01 (Student's t-test)

On observe une inhibition de l'effet de l'aloïsine A par la Brefeldine A (BFA), inhibiteur du trafic vésiculaire ERGIC, ce qui montre que l'aloïsine A induit un réadressage de la protéine delF508-CFTR.

### R3. Cytotoxicité, de l'aloïsine A

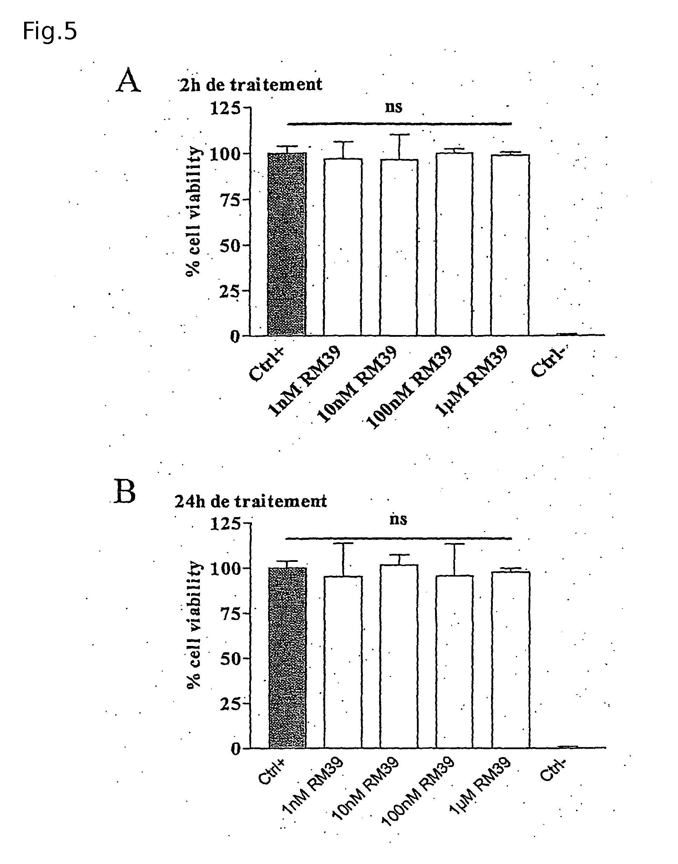

Dans le but de tester la cytotoxicité de l'aloïsine A, des cellules CHO-WT ont été incubées 2 h (figure 5A) ou 24 h (figure 5B) avec différentes concentrations d'aloïsine A avant de subir le test de viabilité cellulaire au MTT. Les résultats montrent que les cellules sont viables pour toutes les concentrations. Cette molécule ne présente donc pas de cytotoxicité cellulaire.

Des expériences menées en efflux d'iodure, en patch clamp ainsi qu'en chambre de Ussing ont montré que l'aloisine A était un activateur des protéine CFTR sauvage et mutées (F508del, G551D, G1349D). Cette molécule affine présente des EC₅₀ variant de 1.5 à 303 nM suivant le mutant testé.

De plus, les expériences effectuées ont démontrés qu'un traitement des cellules CF15 (F508del/F5-08del) par l'aloisine A permettait le réadressage de la protéine mutée F508del CFTR à la membrane.

Des expériences d'efflux d'iodure ont montré que l'aloisine A permet le réadressage de la protéine F508del-CFTR après 2 h de traitement avec une EC₅₀ de 49µM.

Ces résultats démontrent que l'aloisine A est un activateur de CFTR à faible concentration mais que cette molécule peut aussi agir aussi comme chaperonne pharmacologique à forte concentration.

De plus, des études de toxicité de l'aloïsine A, réalisées chez l'animal, ont révélé une toxicité très faible.

Les résultats ci-dessus ont été également vérifiés avec d'autres dérivés de pyrrolo-pyrazines.

### Exemple de formulation

On prépare une solution pour inhalation avec un nébuliseur ampoule à partir de chlorure de sodium, de chlorure de calcium déshydraté et d'eau pour préparations injectables.

L'aloïsine A est ajoutée comme ingrédient actif.

La solution est formulée dans des ampoules de 2,5ml.

Des ampoules renfermant 5, 10 mg ou 20 mg l'aloïsine sont ainsi préparées.

### BIBLIOGRAPHIE

BECQ et al. (1999) Journal of Biological Chemistry 274, 27415-27425.
DORMER et al. (2001) Journal of Cell Science 114; 4073-4081.

## Revendications

1. Utilisation de dérivés de pyrrolo-pyrazines pour fabriquer des médicaments pour le traitement de la mucoviscidose , ces dérivés répondant à la formule (I): 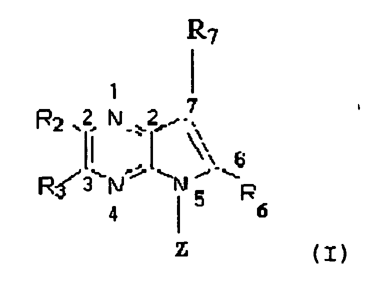 dans laquelle
- R2 et R3 sont identiques ou différents et représentent H, C1-C6 alkyl, ledit alkyl étant une chaîne alkyle droite ou ramifiée, le cas échéant substituée,
- R6 est un cycle aromatique Ar ou un cycloalkyle, le cas échéant substitué, ledit cycloalkyle étant le cas échéant substitué par un groupe aryle qui peut être également substitué,
- R7 est H, C1-C6 alkyl, (alk.)ₙ-hal., CH₂-CH = CH₂, CH₂-cycloalkyl, CH₂-Ar, n est 1-6
- Z est H ou CH₃.

2. Utilisation selon la revendication 1, **caractérisée en ce que** R2 et R3, et/ou Z et/ou R7 sont différents de H.

3. Utilisation selon la revendication 1, **caractérisée en ce que** lesdits dérivés de pyrrolo-pyrazines présentent la formule (II) : 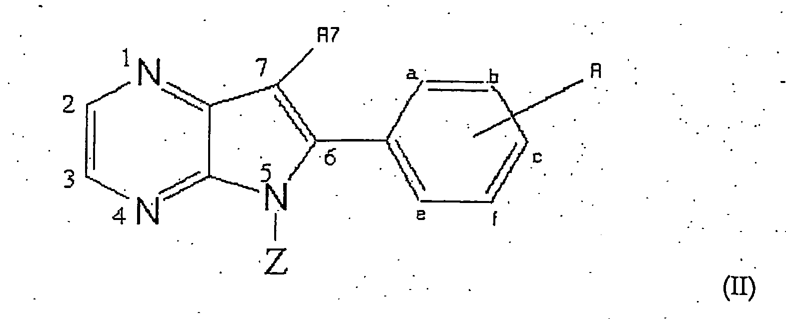 dans laquelle
- le groupe phényl en position 6 est substitué par un, deux ou trois substituants R choisis dans le groupe comprenant :
- H, -OH, alkyl, -O alkyl, hal., -NH₂, -N(H,alkyl), - N(alkyl)₂, -O-SO₂-NH₂, -O-SO₂-N(H, alkyl), -O-SO₂-N(alkyl)₂,-COOH, -COO-alkyl, CONH₂, -CON(H,alkyl), -CON(alkyl)₂,
- R7 est H, alkyl, (alk.)ₙ hal., -CH₂-CH = CH₂, (alk.)ₙ-cycloalkyl, alk.-Ar, et
- Z est H ou CH₃.

4. Utilisation selon la revendication 3, **caractérisée en ce que** Z et/ou R7 sont différents de H.

5. Utilisation de l'aloïsine A de formule (III) 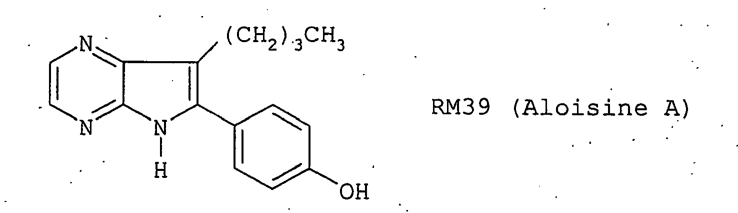 pour fabriquer des médicaments pour le traitement de la mucoviscidose.

6. Utilisation selon l'une quelconque des revendications 1 à 5, **caractérisée en ce que** les médicaments sont préparés pour une administration sous formes de gélules, comprimés, dragées ou capsules.

7. Utilisation selon l'une quelconque des revendications 1 à 5, **caractérisée en ce que** les médicaments sont préparés pour une administration par voie injectable, sous forme de solution.

8. Utilisation selon l'une quelconque des revendication 1 à 5, **caractérisée en ce que** les médicaments sont préparés pour une administration sous forme d'aérosol.

9. Utilisation selon l'une quelconque des revendications 1 à 8, pour le traitement de la mucoviscidose.

## Claims

1. The use of pyrrolopyrazine derivatives for the manufacture of medicaments for the treatment of cystic fibrosis, these derivatives corresponding to formula (I): 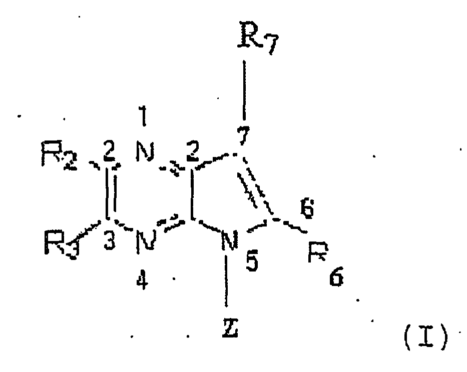 in which
- R2 and R3 are identical or different and represent H, C1-C6 alkyl, said alkyl being a straight or branched alkyl chain, where appropriate substituted,
- R6 is an aromatic ring Ar or a cycloalkyl, where appropriate substituted, said cycloalkyl being where appropriate substituted with an aryl group which may also be substituted,
- R7 is H, C1-C6 alkyl, (alk.)ₙ-hal., CH₂-CH=CH₂, CH₂-cycloalkyl, CH₂-Ar, n is 1-6
- Z is H or CH₃.

2. The use as claimed in claim 1, **characterized in that** R2 and R3, and/or Z and/or R7 are different from H.

3. The use as claimed in claim 1, **characterized in that** said pyrrolopyrazine derivatives have the formula (II): 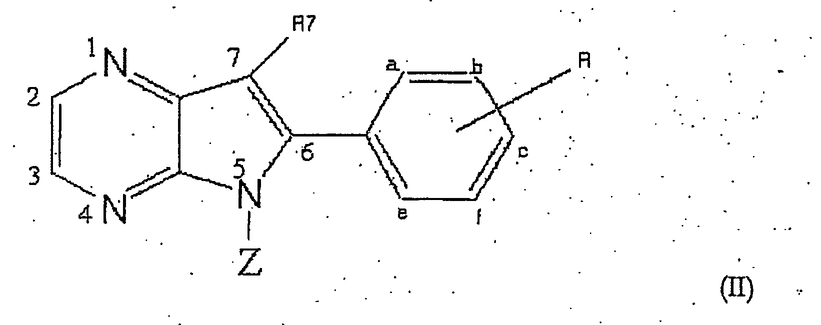 in which
- the phenyl group at the 6-position is substituted with one, two or three substituents R chosen from the group comprising:
- H, -OH, alkyl, -O alkyl, hal., -NH₂, -N(H,alkyl), -N(alkyl)₂, -O-SO₂-NH₂, -O-SO₂N(H,alkyl), -O-SO₂-N(alkyl)₂, -COOH, -COO-alkyl, CONH₂, -CON(H,alkyl), -CON(alkyl)₂,
- R7 is H, alkyl, (alk.)ₙhal., -CH₂CH=CH₂, (alk.)ₙ-cycloalkyl, alk.-Ar, and
- Z is H or CH₃.

4. The use as claimed in claim 3, **characterized in that** Z and/or R7 are different from H.

5. The use of aloisine A of formula (III) 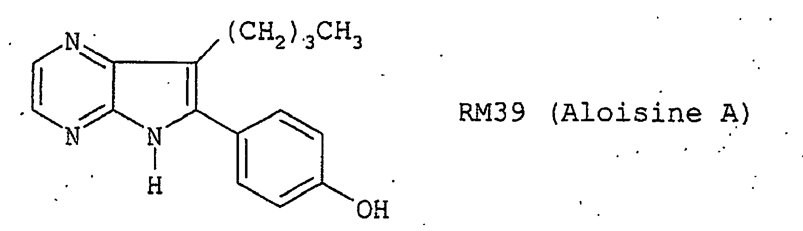 for the manufacture of medicaments for the treatment of cystic fibrosis.

6. The use as claimed in any one of claims 1 to 5, **characterized in that** the medicaments are prepared for administration in the form of gelatin capsules, tablets, sugar-coated tablets or capsules.

7. The use as claimed in any one of claims 1 to 5, **characterized in that** the medicaments are prepared for administration by injection, in the form of a solution.

8. The use as claimed in any one of claims 1 to 5, **characterized in that** the medicaments are prepared for administration in aerosol form.

9. The use as claimed in any one of claims 1 to 8, for the treatment of cystic fibrosis.

## Patentansprüche

1. Verwendung von Pyrrolopyrazinderivaten zur Herstellung von Medikamenten für die Behandlung von Mukoviszidose, wobei die Derivate der Formel (I) entsprechen: 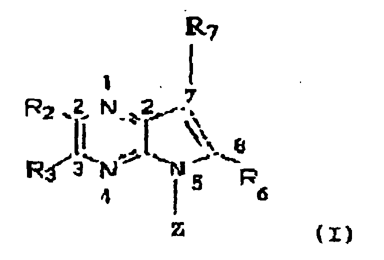 wobei
- R₂ und R₃ gleich oder verschieden sind und für H oder C₁-C₆-Alkyl stehen, wobei das Alkyl eine geradkettige oder verzweigte, gegebenenfalls substituierte Kette ist;
- R₆ ein gegebenenfalls substituierter aromatischer Ring Ar oder ein gegebenenfalls substituiertes Cycloalkyl ist, wobei das Cycloalkyl gegebenenfalls mit einer Arylgruppe, die ebenfalls substituiert sein kann, substituiert ist;
- R₇ = H, C₁-C₆-Alkyl, (alk.)ₙ-Hal, CH₂-CH=CH₂, CH₂-Cycloalkyl, CH₂-Ar ist und n = 1 bis 6 ist;
- Z = H oder CH₃ ist.

2. Verwendung gemäß Anspruch 1, **dadurch gekennzeichnet, dass** R₂ und R₃ und/oder Z und/oder R₇ von H verschieden sind.

3. Verwendung gemäß Anspruch 1, **dadurch gekennzeichnet, dass** die Pyrrolopyrazinderivate die Formel (II) aufweisen: 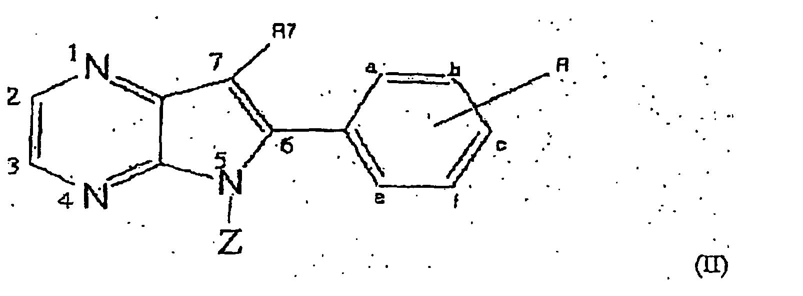 wobei
- die Phenylgruppe auf Position 6 mit einem, zwei oder drei Substituenten R substituiert ist, die aus der Gruppe ausgewählt sind, die Folgendes umfasst:
- H, -OH, Alkyl, -O-alkyl, Hal, -NH₂, -N(H,alkyl), -N(alkyl)₂, -O-SO₂-NH₂, -O-SO₂-N(H,alkyl), -O-SO₂-N(alkyl)₂, -COOH, -COO-alkyl, CONH₂, -CON(H,alkyl), -CON(alkyl)₂;
- R₇ = H, Alkyl, (alk.)ₙ-Hal, -CH₂-CH=CH₂, (alk.)ₙ-Cycloalkyl, alk.-Ar ist und
- Z = H oder CH₃ ist.

4. Verwendung gemäß Anspruch 3, **dadurch gekennzeichnet, dass** Z und/oder R7 von H verschieden sind.

5. Verwendung von Aloisin A der Formel (III) 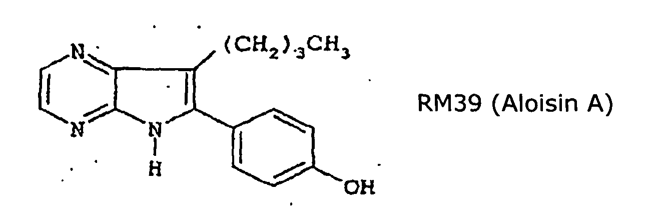 zur Herstellung von Medikamenten zur Behandlung von Mukoviszidose.

6. Verwendung gemäß einem der Ansprüche 1 bis 5, **dadurch gekennzeichnet, dass** die Medikamente für eine Verabreichung in Form von Gelkapseln, Tabletten, Dragees oder Kapseln hergestellt werden.

7. Verwendung gemäß einem der Ansprüche 1 bis 5, **dadurch gekennzeichnet, dass** die Medikamente für eine Verabreichung durch Injektion in Form einer Lösung hergestellt werden.

8. Verwendung gemäß einem der Ansprüche 1 bis 5, **dadurch gekennzeichnet, dass** die Medikamente für eine Verabreichung in Form eines Aerosols hergestellt werden.

9. Verwendung gemäß einem der Ansprüche 1 bis 8 zur Behandlung von Mukoviszidose.
